# EUROPEAN PATENT APPLICATION

(11) **EP 1 462 801 A2**
(43) Date of publication of application: **29.09.2004**
(21) Application number: 04251704.5
(22) Date of filing: 24.03.2004
(51) Int. Cl.: G01N 33/53

(54) **Methods for determining the negative control value for multi-analyte assays**

(30) Priority: 24.03.2003 US 457146 P
(71) Applicant: Tepnel Lifecodes, Stamford, CT 06902 (US)
(72) Inventor: Nordman, David, Metairie, LA 70002 (US); Ray, Bryan, Cheshire, CT 06410 (US); Balazs, Ivan, New Rochelle, NY 10804 (US)
(74) Representative: Perry, Robert Edward

(57) **Abstract**

The negative control value, to be used as a correction to the results obtained when measuring the reaction of a complex biological mixture in a multi-analyte assay, is selected. It is generated using the same sample that is also being analyzed for the presence of analytes of interest.

## Description

### Field of the Invention

This invention relates to methods for determining the negative control value for multi-analyte assays.

### Background of Invention

Various analytical procedures and devices are commonly employed in assays to determine the presence and/or concentration of substances of interest or clinical significance that may be present in biological liquids or other materials. Such substances are commonly termed "analytes" and can include antibodies, antigens, drugs, hormones, etc.

One frequently used assay format is the immunoassay. Immunoassay techniques take advantage of the mechanisms of the immune systems of higher organisms, wherein antibodies are produced in response to the presence of antigens that are pathogenic or foreign to the organisms. These antibodies and antigens, i.e., immunoreactants, are capable of binding with one another, thereby creating a highly specific reaction mechanism that can be used *in vitro* to determine the presence or concentration of a particular antigen or antibody in a biological sample.

There are several known immunoassay methods using immunoreactants, wherein at least one of the immunoreactants is labeled with a detectable component so as to be analytically identifiable. For example, the "sandwich" or "two-site" technique may involve the formation of a ternary complex between an antigen and two antibodies. A convenient method of detecting the presence of analytes in a sample is to provide an unlabeled antibody bound to a solid phase support such that the complex can readily be formed between the unlabeled antibody and the analyte present in the sample. After washing, the solid support is then contacted with labeled antibodies that also bind to the analyte. In this example, the amount of labeled antibody associated with the solid phase is directly proportional to the amount of analyte in the test sample.

In a similar assay format, an antigen may be bound to a solid support, which is then contacted with a test solution that may or may not contain antibodies to the antigen. After washing, the solid support is then contacted with labeled antibodies that bind to any antibodies bound to the analyte. The labeled antibodies can then be detected and their presence directly correlated with the presence of the analyte.

An alternative technique is the "competitive" assay. In one example of a competitive assay, the capture mechanism again may use an antibody attached to an insoluble solid phase, but a labeled analyte competes with the analyte present in the test sample for binding to the immobilized antibody. Similarly, an immobilized analyte can compete with the analyte of interest for a labeled antibody. In these competitive assays, the quantity of captured labeled reagent is inversely proportional to the amount of analyte present in the sample.

A multi-analyte system should involve a means of providing simultaneous analysis of several analytes in a test sample. This analysis should provide results that identify individual analytes and enable the quantitation of each individual analyte in that test sample. A method of multi-analyte analysis is often claimed but the given criteria are generally not both fulfilled.

In a multi-analyte system, a typical substrate contains a plurality of individual test reaction sites each possessing a different binding ligand. The test sample contacts each of the reaction zones and thereafter a variety of detection techniques can be implemented to identify the analyte(s) present. It is preferable that the detection method used enables quantitation of each individual analyte.

Conventional assay methods often use the values obtained from the reaction of a negative control biological material (e.g. sera) with the multi-analyte panel. Often, multiple negative control sera are used. These negative control sera, obtained from several individuals and different from those providing the sample sera, are used to correct for non-specific reaction of sera in the multi-analyte assay. Problems may arise due to the fact that the source of the negative samples is different from that of the unknown sample, resulting in unexpected reactivity.

Thus, conventional methods of determining the negative control value may produce an inaccurate estimate of the true negative value. As a result, for some samples, the multi-analyte assay may produce false positive or false negative results.

### Brief Summary

The present invention provides unique and advantageous methods for selecting the negative control value to be used when correcting the results obtained from measuring the reaction of a complex biological mixture in a multi-analyte assay.

The methods of the subject invention are particularly advantageous because the negative control value(s) is/are generated using the same sample that is also being analyzed for the presence of the analytes of interest.

In one embodiment, the subject invention pertains to the testing of a blood serum sample using indirect detection methods for immunofluorescence or ELISA type assays. This method provides for a negative control value that, when incorporated into sample calculations, reliably determines whether the unknown sera contains specific antibodies against any of the antigens bound to a solid support.

### Detailed Disclosure

The subject invention advantageously provides new and reliable methods for selecting the negative control value(s) to be used in multi-analyte assays. Use of the methods of the subject invention can reduce the incidence of false positives and/or false negatives, particularly in multi-analyte assays conducted on complex biological samples.

Advantageously, in accordance with the practice of the subject invention, the negative control value(s) can be selected from the results obtained only with the sample biological material (e.g. patient sera), without the need for processing negative control material (e.g. normal sera).

In one embodiment, the practice of the subject invention involves utilizing one or more "negative reagents" as the binding ligand in the assay. As used herein, "binding ligand" or "capture reagent" refers to an entity attached to a solid support and which specifically binds to a target analyte, except for the negative control reagent, which is a reagent for which it is known that the sample of interest does not contain a chemical entity that would specifically bind to the reagent. Thus, this negative control reagent could be, for example, an antigen for which it is known that the sample of interest does not contain an antibody that specifically binds to the antigen. For example, a non-human HLA protein could be used as the negative control reagent for a test wherein the sample of interest is a human sample. Preferably, the negative control reagent is an entity that has physical, chemical, and/or antigenic properties in common with at least one capture reagent. The common properties may be, for example, molecular weight, charge, and/or solubility. The negative control reagent may be a homolog, ortholog or other such related molecule to the capture reagent.

It is, of course, ideal to find a negative control reagent that generally behaves the same as the reagents used to detect the specific target analyte(s). However, with any biological assay, there is always the possibility of encountering samples having non-specific variable interactions that pass unrecognized and affect the interpretation of the assay. Thus, to reliably interpret the results of any assay, it is advantageous to not only have within one's repertoire the use of negative control reagents, but multiple techniques for determining negative control values (NCVs).

In a further embodiment, the methods of the subject invention can be used to determine an appropriate NCV even without utilizing either traditional negative control sera or the negative control reagent system as described herein. In this embodiment, reactivity values for one or more low-reacting specific target analytes in a multi-analyte assay are used to establish the NCV(s).

In an embodiment specifically exemplified herein, the methods of the subject invention can be applied to the testing of a blood serum sample using indirect detection methods for immunofluorescence or ELISA type assays. This method provides for identification of a NCV that, when incorporated into sample calculations, reliably determines whether an unknown sera contains specific antibodies against any of the capture reagents bound to a solid support. The solid support may be, for example, beads, wells, membranes, or microarrays.

In one specific embodiment of the subject invention, the panel of binding ligands includes, in addition to the capture reagents for the specific target analytes, one or more non-human proteins or other compounds to neutralize possible charge or chemical interactions that may occur with the solid support. Examples of this type of compound include proteins such as albumin or casein or chemicals that bind or react with the solid support; for example, if the solid support is a carboxylated plastics, it is possible to use chemicals containing amino groups such as ethanolamine or tris (hydroxymethyl) aminomethane.

Following are Examples that illustrate procedures for practising the invention. These examples should not be construed as limiting. All percentages are by weight and all solvent mixture proportions are by volume unless otherwise noted.

### Example 1 - Screening for anti-HLA antibodies in human sera

One or more Human Leukocyte Antigens (HLAs) are attached to a solid surface (e.g. multiple wells of a microtiter plate, or different color or size beads) creating a panel of HLAs. This panel of multiple wells or beads also includes negative controls consisting of one or more wells or beads with molecules that are not HLAs. Patient sera is added to all the wells, or mixed with the different beads, and incubated. After washing, the presence of anti-HLA antibodies can be measured by reaction to a labeled anti-immunoglobulin antibody.

Conventional methods produce a negative control by performing several assays using sera from individuals that do not contain anti-HLA antibodies. This is done in order to estimate the amount of non-specific reactivity between sera and capture reagents (antigens) on the solid support. Typically, the median or average values are used to establish a negative control value (NCV).

US-A-6046013 describes a typical process for obtaining the negative control value using a similar type of assay.

It would be extremely rare for an individual to have anti-HLA antibodies against every antigen in the panel; therefore, in one embodiment of the subject invention, the antibody/antigen complex with the lowest signal can be considered the raw NCV. There may be several analytes with similar values; however, since additional calculations will be performed to compensate for assay variability, in one embodiment it is sufficient to use the lowest value. Alternatively, an average of multiple low values can be used. These "low" values would be clearly distinguishable from higher values that reflect a positive result for a particular analyte. The "low" values would, for example, be within 20% of the lowest value and, preferably, within 10% of the lowest value.

The raw NCV may be further adjusted by subtracting the background value (BGV) (i.e. inherent reactivity of the solid support) to obtain a calculated NCV.

The BGV reflects the inherent reactivity of each capture reagent, with the detection antibody. This can be measured by adding water or buffer instead of serum sample to the multi-analyte panel. The BGV may be the same or vary significantly between capture reagents. If there are large differences between the BGVs, each analyte measurement can be corrected by subtracting its own BGV. Alternatively, it is possible to use the same median BGV or average BGV for all analytes.

Another way to determine the NCV for non-specific binding of sample sera to the solid support is by evaluating the reactivity of the sample with one or more non-human HLA-like proteins. In a specific embodiment, leukocyte antigens from non-primates can be attached to a solid support to create non-specific interactions similar to those produced by HLA.

### Example 2 - Determination of Negative Control Value

Table 1 shows how to identify the NCV in accordance with one embodiment of the methods of the subject invention.

An "analyte panel" was created by attaching 17 capture reagents to a solid support. A positive (#16) and negative (#17) control were included on the panel.

The sample and water were contacted with the analyte panel (the panel of capture reagents) and subsequently rinsed to wash away unbound molecules. A label was added to detect the attached molecules. The label was detected and the intensity or amount (e.g., absorbance, fluorescence) recorded as shown in Table 1.

The value recorded from each reaction of water and capture reagent is an individual BGV. The average BGV of 26 could be used, but the BGVs had a broad range; therefore, each individual BGV was subtracted from the raw value recorded from each reaction of the sample. These adjusted values were compared to the negative control (#17).

A review of Table 1 reveals that the lowest adjusted value was the reaction of the sample with capture reagent #4 (123) instead of the negative control #17 (530). Therefore, the value for analyte #4 can be used as the negative control instead of the value for analyte #17 Otherwise other analytes would end up as false negatives.

In this example, the raw NCV is the raw value of the sample reacted with capture reagent #4 (158). The calculated NVC is identified by subtracting the raw value of water reacted with capture reagent for analyte #4 (35) from the raw value of the sample reacted with the capture reagent for analyte #4 (158). Therefore, the NCV is 123.

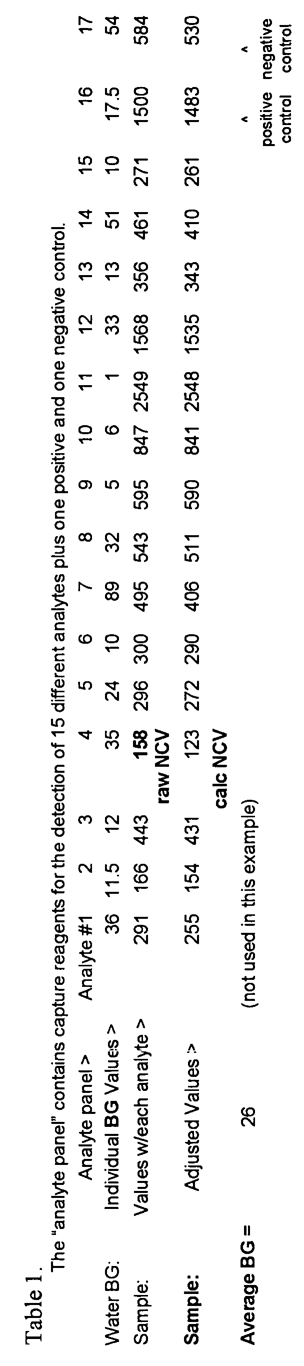

### Example 3 - Determination of Negative Control Value

Table 2 shows another example of how to identify the NCV in accordance with the methods of the subject invention.

The same panel for the detection of 17 analytes used in Example 2 was used in this example. This panel included a positive (#16) and a negative (#17) control. However, a different sample was used in this example. In this case, the lowest adjusted value was that obtained from the reaction of the sample with the negative control #17. Therefore, in this case, the adjusted value for #17 would be used as the NCV. Otherwise, many analytes would end up as false negatives.

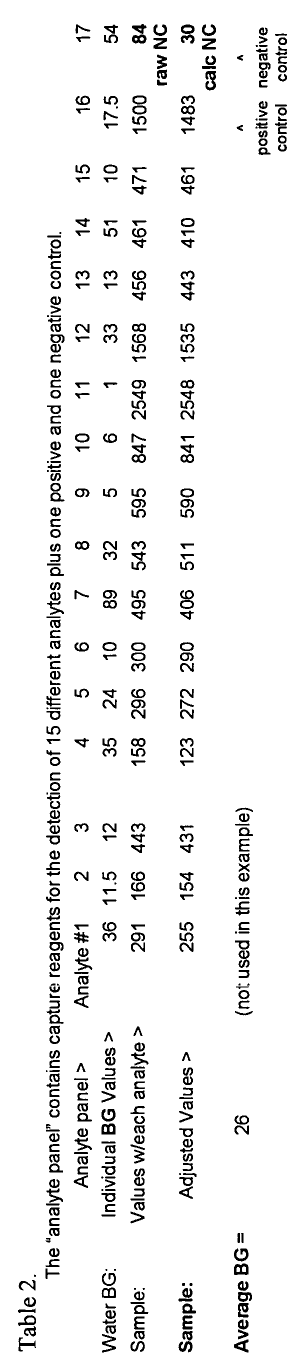

### Example 4

Conventional methods utilize a single "negative reagent" to determine the level of non-specific interaction of a sample in a multi-analyte assay. This is done in order to estimate the amount of non-specific reactivity between sera and the multi-analyte solid support. However, with any biological assay, there is always the possibility of encountering samples having non-specific variable interactions. These interactions may occur with one "negative reagent" but not with a chemically different "negative reagent". Thus, it is advantageous to have within one's repertoire multiple types of "negative reagent" controls (NRC).

In one preferred embodiment, two or more different NRC are included in a multi-analyte panel. After the assay with the sample sera, a calculated NC value is obtained for each NRC. The calculated NC value for each NRC is used separately to evaluate the reactivity of the sample against the analytes in the panel. This produces as many sets of results as the number of NRCs included in the multi-analyte panel. The comparison of these sets shows which analytes are consistently positive or negative with the different NRCs.

For example, if four independent NRCs are included in the multi-analyte panel, four sets of results are generated. By counting the number of times that an analyte generates the same type of results in the four sets, it is possible to determine the concordance or consistency of the results.

One method to interpret the results is to count the number of times an analyte is positive in the four sets. if the analyte is positive m 3 or 4 sets of results, it would be considered Positive. If it was positive in 2 sets it would be considered as Tentative or Doubtful Positive. If it was positive in 1 set it would be considered as Tentative or Doubtful Negative. If it was not positive in any of the sets in would be considered Negative.

### Example 5 - Effect of Multiple Control beads on Results from an Antibody Screening Assay

A serum sample was assayed using the Lifematch Antibody Screen assay (Tepnel Lifecodes, Stamford, CT), designed for use with a Luminex 100 fluoranalyzer, which was modified to include three negative control beads. This product consists of one bead population with HLA Class I antigens attached, a second population with Class II antigens attached, a third population with Human IgG that serves as a positive control for the detection reagent, a fourth population that contains an analyte not related to HLA that serves as negative control (CON1), a fifth population that contains a different analyte as a second negative control (CON2) and a sixth population that contains yet a different analyte that serves as a third negative control (CON3).

As described below, the median fluorescent intensity (MFI) for each antigen-containing bead (i.e., Class I or Class II beads) is divided by the MFI of each negative control bead yielding 3 quotients. From each quotient is subtracted a background adjustment factor (BAF) that is provided by the kit manufacturer. This results, for each analyte, in three *adjusted values* (AdjVal). If any adjusted value is greater than zero, a positive reactivity for an antigen-containing bead is indicated. Table 3 illustrates the results obtained for this example assay.

**Table 3**

| Bead | MFI | BAF | AdjVal1 | Adj Val2 | AdjVal-3 | Result |
|---|---|---|---|---|---|---|
| | | | (a) | (b) | (c) | |
| Class I | 10,137.5 | | 52.92 | 29.94 | 28.97 | Positive |
| CONI | 182 | 2.785 | | | | |
| CON2 | 311.5 | 2608 | | | | |
| CON3 | 323.5 | 2.367 | | | | |
| Class II | 652 | | 0.51 | -0.92 | -076 | Positive |
| CONI | 182 | 3.068 | | | | |
| CON2 | 311.5 | 3.009 | | | | |
| CON3 | 323.5 | 2.774 | | | | |
| (a) AdjVal1 = (MFI/CON1)- BAF_{CON 1}; | | | | | | |
| (b) Adj Val2 = (MFI/CON2) - BAF_{CON 2}; | | | | | | |
| (c) Adj Val3 = (MFI/CON3) - BAF_{CON 3} | | | | | | |

For Class I, a positive reaction is indicated by all three adjusted values, whereas for Class II, a positive reaction is indicated for only one adjusted value. Because two of the negative control beads (CON2 & CON3) show a higher background than CON1, they yield a false negative result.

As shown in this example, if only CON2 or CON3 were present in the assay, this sample would have been incorrectly assigned as having no antibody toward Class II antigens. Thus, inclusion of multiple negative control beads reduces the likelihood of a false negative assignment.

### Example 6 - Application of the lowest signal analyte as Negative Control to compensate for effect of high background

This example illustrates the effect of employing the lowest signal analyte as negative control for the analysis of a sample that has a high background with the Negative Control Bead.

A serum sample containing antibodies toward HLA-A2 and HLA-A68 antigens was assayed using the Lifematch Class I ID kit (Tepnel Lifecodes, Stamford, CT) and analyzed with a Luminex 100 fluoroanalyzer. The assay was performed according to the product insert. Briefly, the product contains a Negative Control Bead, a Positive Control Bead and a series of beads that contain various combinations of antigens. The assay comprises 1) mixing a serum sample with the beads, 2) washing away unbound sera, 3) adding a label that binds to the captured HLA-antibodies and 4) detecting the amount of label bound to each bead with the Luminex 100. The amount of label bound is reported as median fluorescence intensities (MFI).

For this example, the MFI values for each analyte is shown in Table 4. The results show that this sample has a high non-specific interaction with the Negative Control (CON1) bead.

In one method of analysis, the MFI for each antigen-containing bead is divided by the MFI of CON1. From this quotient is subtracted a background adjustment factor (BAF) that is provided by the kit manufacturer. A resultant greater than zero indicates a positive reactivity for an antigen-containing bead.

The use of this calculation for CON1 results in the classification of only 2 analyte containing beads as positive. As such, the sample may be erroneously classified as antibody negative. In addition, the concordance between the positive beads and a particular analyte (e g. HLA-A antigen) in the bead is insufficient for antibody identification.

An alternative method for calculating a positive reaction employs an antigen-containing bead as a negative control. For most assays, a serum sample will not have a positive reaction with all antigen-containing beads. Thus, the antigen-containing bead that has the lowest MFI reading can be employed as a negative control to calculate an adjusted value (AdjVal2).

To determine reactivity, the same calculation is applied to the antigen-containing beads except that the MFI of CON1 bead is replaced by the MFI of the lowest value bead. In this same example, using the MFI of the lowest value bead (#156), results in the classification of 26 analyte containing beads, as positive (AdjVal2; Table 4). An examination of the analytes present in those beads shows 100% correlation with the presence of HLA-A2 and HLA-A68 on the beads. Therefore, in samples with high values for CON1, the analysis of the results using the lowest value analyte containing bead, allows for the correct identification of the antibodies present in the sample.

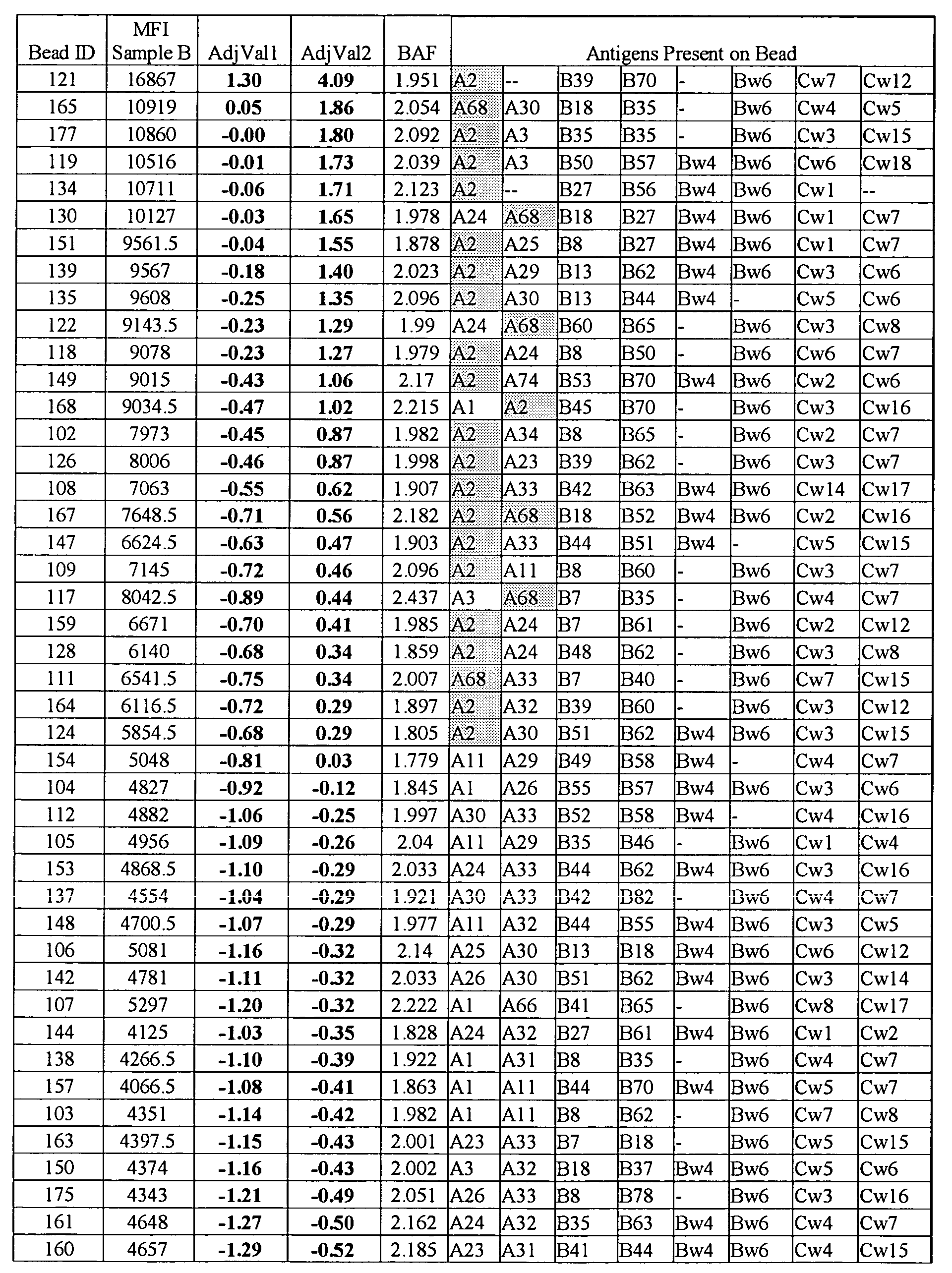

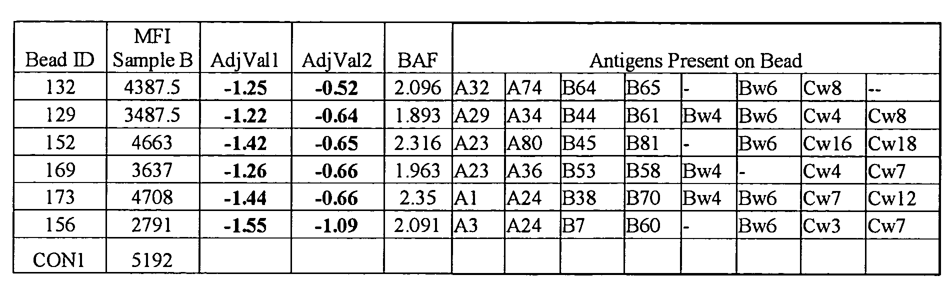

## Claims

1. A multi-analyte assay wherein capture reagents are used to detect analytes in a sample, wherein a negative control value used in calculations to determine the presence or absence of an analyte in the sample is determined by a method comprising contacting the sample with at least one negative control reagent attached to a solid support, wherein the negative control reagent is of similar molecular structure to at least one capture reagent, but which does not specifically bind to any entity or analyte in the sample, and wherein the assay further comprises quantifying the extent of any reaction between the sample and the negative control reagent, and recording a value representing the extent of this reaction.

2. A multi-analyte assay wherein a negative control value used in calculations to determine the presence or absence of an analyte in a sample is determined by a method comprising contacting the sample with, in addition to a plurality of capture reagents, at least two negative control reagents attached to at least two solid supports or different areas of a solid support, wherein the at least two negative control reagents are entities that cannot specifically bind to any entity or analyte in the sample, and wherein the assay further comprises quantifying the extent of any reaction between the sample and the negative control reagents, and recording the values representing the extent of these reactions, wherein the negative control value is the average of said values.

3. An assay according to claim 2, wherein a negative control value is selected and a corresponding calculation is performed based on values obtained for each of the at least two negative control reagents and the extent of any sample reaction is based on the results of one or more of the calculations.

4. An assay according to claim 2 or claim 3, wherein at least three negative control reagents are used.

5. An assay according to any preceding claim, wherein the or at least one of the negative control reagents is an entity that has physical and/or chemical properties in common with at least one capture reagent.

6. An assay according to claim 5, wherein the physical and/or chemical properties include molecular weight, charge, tertiary structure, conformation and/or solubility.

7. An assay according to any preceding claim, wherein the sample is obtained from a human and at least one negative control reagent is a non-human protein that does not specifically bind with any analytes in the sample.

8. An assay according to any preceding claim, wherein the or each solid support is selected from beads, wells, membranes and microarrays.

9. An assay according to any preceding claim, further comprising the step of subtracting from the negative control value a background value representing the reaction between a detection molecule and at least one capture reagent attached to the solid support.

10. A multi-analyte assay wherein a negative control value used in calculations to determine the presence or absence of an analyte in a sample is determined by a method comprising contacting the sample with a plurality of capture reagents, measuring the reaction of the sample with each of the capture reagents and using, as a negative control value, the lowest of the measured reactions or an average of low reactions.

11. An assay according to any preceding claim, wherein the or at least one analyte is an antibody or antigen.

12. An assay according to any preceding claim, wherein the sample is serum, tissue or urine.

13. An assay according to any preceding claim, which comprises determining the presence or absence of an analyte in the sample by comparison of the results of reaction with a capture reagent and the negative control.
